# EUROPÄISCHE PATENTSCHRIFT

(11) **EP 0 756 471 B1**
(45) Veröffentlichungstag und Bekanntmachung des Hinweises auf die Patenterteilung: **04.12.2002**
(21) Anmeldenummer: 96902873.7
(22) Anmeldetag: 20.02.1996
(51) Int. Cl.: A61B 1/00, A61B 19/00

(54) **VORRICHTUNG ZUR UNTERSUCHUNG VON HOHLRÄUMEN MIT EINEM ENDOSKOP**
DEVICE FOR EXAMINING CAVITIES WITH AN ENDOSCOPE
DISPOSITIF D'EXAMEN DE CAVITES AU MOYEN D'UN ENDOSCOPE

(30) Priorität: 20.02.1995 DE 19505774; 20.02.1995 DE 19505775
(43) Veröffentlichungstag der Anmeldung: 05.02.1997
(73) Patentinhaber: Storz, Sybill, 78532 Tuttlingen (DE)
(72) Erfinder: Storz, Karl Dr., 78532 Tuttlingen (DE)
(74) Vertreter: Münich, Wilhelm, Dr.
(86) Internationale Anmeldenummer: DE9600264
(87) Internationale Veröffentlichungsnummer: WO96025873

(56) Entgegenhaltungen:
- WO-A-89/02202
- DE-A- 2 507 346
- DE-U- 9 411 556
- FR-A- 2 433 331
- US-A- 4 991 565
- US-A- 5 039 198

## Beschreibung

### Technisches Gebiet

Die Erfindung bezieht sich auf eine Vorrichtung zur Untersuchung von Hohlräumen insbesondere im menschlichen oder tierischen Körper gemäß dem Oberbegriff des Patentanspruchs 1.

### Stand der Technik

Eine Vorrichtung zur Untersuchung von Hohlräumen der im Oberbegriff des Patentanspruchs 1 vorausgesetzten Art ist aus der US-A-5 039 198 bekannt:

Die bekannte gattungsgemäße Vorrichtung ist für die stereoskopische Betrachtung von Hohlräumen insbesondere bei mikrochirurgischen Operationen gedacht, und weist ein Spezialendoskop auf, das aus zwei voneinander unabhängigen Endoskopteilen besteht. Jeder Endoskopteil ist praktisch ein vollständiges Endoskop und verfügt in bekannter Weise über einen Lichtleiter zur Beleuchtung des Hohlraums sowie über ein Objektiv zur Abbildung des Objektfeldes des Hohlraums, einen Bildweiterleiter für das Bild des Objektivs und ein Okular zur Betrachtung des weitergeleiteten Bilds. Ferner ist als Kopfhalter ein Kopfband vorgesehen, an dem die Okulare der beiden Endoskopteile derart angebracht sind, daß die Okulare vor dem linken bzw. rechten Auge der Untersuchungsperson positioniert sind. Zur Verbreiterung der Stereobasis sind die distalen Enden der beiden Endoskopteile an einem Träger gehalten, der für den notwendigen Abstand der Objektive der beiden Endoskopteile sorgt.

Allerdings erschwert der Träger das Einführen des Stereoendoskops in den Hohlraum, in dem der Beobachtungs- bzw. Behandlungsvorgang durchgeführt werden soll.

Vor allem aber ist die aus der US-A-5 039 198 bekannte Vorrichtung aufgrund der Verwendung eines aus zwei getrennten Endoskopen bestehenden Spezialendoskops sehr aufwendig.

Die vorstehend genannten Nachteile dürften der Grund dafür sein, daß keine Vorrichtung zur Untersuchung von Hohlräumen kommerziell angeboten wird, die entsprechend der US-A-5 039 198 aufgebaut ist.

Ferner ist es bekannt, herkömmliche Endoskope an einer mechanischen Halterung, wie beispielsweise einem Ständer oder einem Stativ zu halten, damit die Untersuchungsperson beide Hände für den Behandlungsvorgang frei hat. Diese Halterung kann so ausgebildet sein, daß sie gleichzeitig einen gewissen Schutz gegen eine Beschädigung des Endoskops und insbesondere eines dünnen Endoskops - beispielsweise durch eine ungewollte Berührung des Bildweiterleiterteils - bietet.

Die bekannten mechanischen Halterungen haben jedoch den Nachteil, daß die Untersuchungsperson ohne Verstellung der Halterung den Kopf nicht bewegen kann, ohne den Blickkontakt zu dem Untersuchungsobjekt zu verlieren.

Dies gilt insbesondere dann, wenn das Endoskop ein ultradünnes Endoskop ist, d.h. ein Endoskop mit einem Durchmesser von etwa 1 bis 2 mm und weniger und entsprechend kleiner Apertur. Derartige Endoskope werden für eine Reihe von Untersuchungsvorgängen in der Technik oder am menschlichen bzw. tierischen Körper benötigt.

Ultradünne Endoskope sind in der Regel flexible Endoskope, in Sonderfällen werden sie jedoch auch als sogenannte starre Endoskope gefertigt.

In jedem Falle müssen ultradünne Endoskope aufgrund ihres geringen Durchmessers und der hierdurch nur geringen Stabilität mit größter Sorgfalt behandelt werden. Eine Halterung für ultradünne Endoskope, durch die die Beschädigungsgefahr deutlich herabgesetzt wird, ist bislang nicht bekannt.

### Darstellung der Erfindung

Der Erfindung liegt die Aufgabe zugrunde, eine Vorrichtung zur Untersuchung von Hohlräumen insbesondere im menschlichen oder tierischen Körper mit einem Endoskop anzugeben, die ohne Verwendung eines teueren Spezialendoskops mit normalen, handelsüblichen Endoskopen die Beobachtung des Hohlraums erlaubt, ohne daß die Untersuchungsperson das Endoskop in der Hand halten muß, wobei die Untersuchungsperson eine gewisse Bewegungsfreiheit für ihren Kopf hat, ohne den Blickkontakt zum Okularbild zu verlieren.

Eine erfindungsgemäße Lösung dieser Aufgabe ist im den Patentansprüchen 1 und 2 angegeben. Weiterbildungen der Erfindung sind Gegenstand der Ansprüche 3 folgende.

Die erfindungsgemäße Vorrichtung weist einen Kopfhalter auf, an dem das Okular des (in herkömmlicher Weise ausgebildeten) Endoskops derart angebracht ist, daß es vor einem Auge der Untersuchungsperson positioniert ist. Durch den Kopfhalter macht das Endoskop die Bewegung des Kopfes der Untersuchungsperson mit, so daß der Blickkontakt ins Okular auch bei kleiner Apertur nicht verloren geht.
Bei der erfindungsgemäßen Ausgestaltung der Vorrichtung ist an dem Kopfhalter ein Betrachtungssystem angebracht, das das Okularbild für die Untersuchungsperson vergrößert. Der Okulartrichter des Endoskops ist hierzu monokular mit dem einem Strahlengang des Betrachtungssystems derart verbunden, daß das Okularbild für die Untersuchungsperson vergrößert wird. Diese Vergrößerung des Okularbildes ist insbesondere deshalb von Vorteil, weil das Okularbild beispielsweise von sehr dünnen (ultradünnen) Endoskopen verglichen mit normalen Endoskopen klein ist.

Erfindungsgemäß ist also ein einziges Endoskop vorgesehen, dessen Okulartrichter monokular mit einem Strahlengang des binokularen Betrachtungssystems verbunden ist. Der scheinbare Nachteil der erfindungsgemäßen Vorrichtung, nämlich daß eine Betrachtung des Hohlraums nicht mit beiden, sondern nur mit einem Auge möglich ist, ist in der Praxis sogar ein Vorteil:
Zum einen sind es beispielsweise Ärzte gewohnt, Okularbilder von Endoskopen nur mit einem Auge zu betrachten. Zum anderen erlaubt es der monokulare Einblick in das Endoskop, mit dem anderen Auge die Umgebung, also beispielsweise den Einstich des Endoskops in den menschlichen Körper zu kontrollieren, ohne daß der "Blick vom Okular abgewendet" werden müßte.

Vor allem aber erlaubt die erfindungsgemäße Vorrichtung die Verwendung herkömmlicher und damit in den meisten Fällen bereits vorhandener Endoskope.
Der Kopfhalter kann dabei in ähnlicher oder gleicher Weise ausgebildet sein wie Kopfbänder, die als Halterung z.B. für Binokularlupen auch aus dem Bereich der Medizintechnik, wie beispielsweise dem Hals-Nasen-Ohren-Bereich bekannt sind. Insbesondere kann das Kopfband (Anspruch 4) beispielsweise aus an der Stirn und am Hinterkopf anliegenden Teilen oder einem ringförmigen Teil und einem in seiner Länge einstellbaren elastischen Band bestehen, so daß das "Kopfband" in Art einer "Haube" auf den Kopf der Untersuchungsperson aufgesetzt werden kann. Selbstverständlich sind aber auch andere Ausgestaltungen des Kopfbandes möglich. Bezüglich der Ausgestaltung von Kopfbändern wird im übrigen auch auf die US-A-5 039 198 verwiesen.

Weiterhin kann gemäß Anspruch 3 der Kopfhalter in Art einer Brillenfassung - gegebenenfalls mit zusätzlichem Kopfband - ausgebildet sein. Dabei kann der Kopfhalter bevorzugt ähnlich einer sogenannten Meßbrillenfassung aufgebaut sein, wie sie beispielsweise von Augenoptikern für die subjektive Refraktionsbestimmung eingesetzt werden. Meßbrillenfassungen erlauben nämlich eine problemlose Anpassung an die verschiedensten Kopfformen und sind darüberhinaus darauf ausgelegt, vergleichsweise schwere Teile zu halten, ohne daß es zu Unbequemlichkeiten für den Brillenträger kommt, oder daß die Meßbrille auf dem Nasenrücken rutscht. Insbesondere können derartige Brillenfassungen leicht durch Veränderung des Brückenabstandes, der Bügellänge, der Abstützflächen auf dem Nasenrücken etc. an unterschiedliche Benutzer angepaßt werden, so daß die erfindungsgemäße Vorrichtung von den verschiedensten Benutzern benutzt werden kann

Da Meß-Brillenfassungen von einer Reihe von Herstellern angeboten werden, muß an dieser Stelle nicht näher auf ihre genaue Ausbildung eingegangen werden. Selbstverständlich ist aber auch der Einsatz anderer Brillenfassungen möglich.

Für die Erfindung ist bezüglich der Ausgestaltung des Kopfhalters grundsätzlich nur von Bedeutung, daß er eine sichere und rutschfreie Anbringung am Kopf der Untersuchungsperson erlaubt.

Das Betrachtungssystem kann gemäß Anspruch 5 beispielsweise eine Brillenfassung mit -zur Vergrößerung des Okularbildes und/oder zur Korrektion von Sehfehlern geeigneten Gläsern, eine Binokularlupe oder ein Fernrohr und insbesondere ein Prismenfernrohr sein, dessen objektseitige Schnittweite auf den Anwendungsfall abgestimmt ist.

Als Binokularlupe kann jede an sich bekannte Binokularlupe, wie sie beispielsweise im Bereich der Medizintechnik verwendet werden, eingesetzt werden.

Welches Betrachtungssystem eingesetzt wird, wird u.a. vom Anwendungsfall und/oder der benötigten Vergrößerung des Okularbildes abhängen:

Die "kumulierte" Vergrößerung der Beobachtungseinrichtung und des Endoskops ist auch für die Durchführung komplizierter Eingriffe ausreichend, so daß aufgrund der Verwendung einer erfindungsgemäßen Vorrichtung häufig auf ein Operationsmikroskop verzichtet werden kann, das in der Anschaffung um ein Mehrfaches teurer ist als eine Binokularlupe bzw. ein entsprechendes Fernrohr. Die Verwendung eines Fernrohrs bzw. einer Binokularlupe hat dabei den besonderen Vorteil, daß nicht nur die Umgebung, sondern auch das Okularbild stark vergrößert wird. Damit ist es möglich, sogar mikrochirurgische Eingriffe unter Beobachtung mit einem Endoskop auszuführen.

Beispielsweise bei der Durchführung von Eingriffen am Auge ist die Vergrößerung einer Binokularlupe oder eines Fernrohrs völlig ausreichend, um das Auge und insbesondere die Umgebung des Einstichs des Endoskops während des Untersuchungs- bzw. Behandlungsvorgangs zu beobachten. Andererseits beschränkt sich die Bedienung eines Fernrohrs bzw. einer Binokularlupe praktisch auf die Einstellung der Pupillendistanz und des zu beobachtenden Objektfeldes. Im Vergleich hierzu ist die Bedienung eines Operationsmikroskops sehr kompliziert.

Bei der Verwendung einer Brillenfassung ist es beispielsweise möglich, den Gläserring, der nicht mit dem Okulartrichter des Endoskops über eine Kupplung verbunden wird, mit einem Brillenglas ohne optische Wirkung oder mit einem Brillenglas zu versehen, das eine optische Wirkung (sphärische und zylindrische Wirkung) hat, die Sehfehler der Untersuchungsperson für die Betrachtung der Umgebung - insbesondere unter Berücksichtigung des jeweiligen "Arbeitsabstandes" - korrigiert. Selbstverständlich ist es aber auch möglich, das Brillenglas, durch das in das Okular geblickt wird, zusätzlich zur Lupenwirkung mit einer Wirkung zur Korrektur von Sehfehlern unter Berücksichtigung des Arbeitsabstandes bei Betrachtung des Okularbildes zu versehen. Die Verwendung einer Brillenfassung hat - wie bereits ausgeführt - den Vorteil, daß vergleichsweise kostengünstige Brillenfassungen handelsüblich angeboten werden, die durch eine gute Anpassung an die Physiognomie der jeweiligen Untersuchungsperson eine hervorragende Gewichtsentlastung ermöglichen.

Die Gewichtsentlastung wird dadurch verbessert, daß sich gemäß Anspruch 11 der Okularteil des Endoskops auf dem Nasenrücken der Untersuchungsperson abstützt. Hierzu ist es nach den Ansprüchen 9 und 10 bevorzugt, wenn das Okular des Endoskops mit dem Betrachtungssystem über ein Strahl-Umlenkelement verbunden ist, das den Strahlengang um 90° umlenkt.

Die Abstützung des Okularteils auf dem Nasenrücken kann beispielsweise mit einem modifizierten Nasensattel erfolgen, der an der Brillenfassung angebracht ist und eine Aufnahme für den Okularteil aufweist.

Die Verbindung zwischen dem Okular des Endoskops und dem (monokularen) Strahlengang des jeweils vorgesehenen Betrachtungssystems kann auf die verschiedensten Arten erfolgen:

Beispielsweise ist es gemäß Anspruch 8 möglich, das Okular ohne zwischengeschaltetes optisches System beispielsweise über eine tellerförmige Kupplung, wie sie auch für das Anflanschen von Videokameras an Endoskope verwendet wird, mit dem Betrachtungssystem zu verbinden. Die Kupplung kann dabei so ausgebildet sein, daß der Okulartrichter gegenüber dem Betrachtungssystem im angekuppelten Zustand gedreht werden kann.

Weiterhin ist es möglich, das Okular mit dem Betrachtungssystem über eine Gliederoptik zu verbinden (Anspruch 2). Derartige Gliederoptiken sind im Bereich der Endoskopie beispielsweise aus der DE 25 07 346 C2 bekannt, so daß auf die Ausbildung derartiger Optiken an dieser Stelle nicht weiter einzugehen ist. Die Verbindung zwischen dem Okular und der Gliederoptik sowie zwischen der Gliederoptik und dem Betrachtungssystem kann wiederum in an sich bekannter Weise erfolgen.

Durch die Verwendung einer Gliederoptik kann die Beobachtungsoptik hin- und herbewegt werden, ohne daß das Endoskop mitbewegt wird, oder der Einblick in das Okular verloren gehen würde. Bei einer weiteren bevorzugten Ausführungsform umgibt eine Spirale die Gliederoptik. Dabei können das bzw. die Lichtleitkabel in der Spirale geführt sein (Anspruch 3).

Anstelle oder zusätzlich zur Gliederoptik kann gemäß Anspruch 12 ein optisches System vorgesehen werden, das beispielsweise durch Einschwenken eines Umlenkprismas in den Strahlengang vor einer oder beider Austrittsöffnungen ein Umschalten zwischen binokularer Beobachtung der Umgebung und (monokularer) Betrachtung des Okularbildes des Endoskops ermöglicht. Bei dieser Anordnung kann auch durch einen geeigneten Strahlteiler ein binokularer Einblick in das Okular des Endoskops realisiert werden, da durch das Ausschwenken eines oder beider Umlenkprismen weiterhin die Möglichkeit besteht, mit einem oder beiden Strahlengängen der Lupe das Umfeld des Einstichs zu beobachten.

Bevorzugt kann der erfindungsgemäß vorgesehene Kopfträger als Träger zusätzlicher bzw. ergänzender Einrichtungen verwendet werden:

So ist es möglich, an dem Kopfband, der Brillenfassung und/oder dem Betrachtungssystem eine Beleuchtungseinrichtung anzubringen, die die Umgebung beleuchtet (Anspruch 13). Damit hat die Untersuchungsperson die Möglichkeit, mit dem einen Auge den Hohlraum zu beobachten, in den das Endoskop eingesetzt ist. Ohne das Kopfband mit dem daran angebrachten Endoskop absetzen zu müssen, kann sie dann die Umgebung, beispielsweise den "Einstich" des Endoskops in einen menschlichen oder tierischen Körper, inspizieren. Diese Beleuchtungseinrichtung kann bei Verwendung eines Binokular-Betrachtungssystems insbesondere zwischen den beiden Strahlengängen des Betrachtungssystems angeordnet sein (Anspruch 14).

Wie bereits erwähnt, kann das Kopfband auch als Träger weiterer Einrichtungen dienen:

So ist es bevorzugt, wenn das Lichtleit-Kabel für das ultradünne Endoskop und/oder der Lichtleiter für die Beleuchtungseinrichtung am Kopfträger geführt sind (Ansprüche 16 und 17). Hierdurch ist gewährleistet, daß sich die Bedienungsperson nicht in Kabeln "verfängt".

Die Verwendung eines Kopfträgers ist bislang für sehr dünne (ultradünne) Endoskope offensichtlich wegen der bei diesen Endoskopen bestehenden Bruchgefahr nicht in Betracht gezogen worden. Versuche des Erfinders haben jedoch gezeigt, daß die Gefahr einer Beschädigung des Endoskops bei einer Anbringung an einem Kopfträger wesentlich geringer als bei der Verwendung einer starren mechanischen Halterung ist, die unabhängig von der Untersuchungsperson positioniert wird.

Wie bereits ausgeführt, sind ultradünne Endoskop in der Regel flexible Endoskope. Da diese Endoskope sehr bruchgefährdet sind, und somit mit größter Sorgfalt behandelt werden müssen, ist bei einer bevorzugten Ausgestaltung der Erfindung eine Schutzhülse für das Endoskop vorgesehen, aus der das Endoskop herausziehbar ist (Anspruch 23). Diese Schutzhülse ist bei einer weiteren im Anspruch 24 angegebenen Ausgestaltung der Erfindung an der Kleidung der Untersuchungsperson beispielsweise mit einer Klammer befestigbar. Durch diese Schutzhülse, die insbesondere spiralförmig ausgestaltet sein kann, ist das Endoskop bei Nichtbenutzung geschützt. Da es aus der Schutzhülse immer nur soweit herausgezogen wird, wie es für den jeweiligen Untersuchungsvorgang benötigt wird, wird die Gefahr einer Beschädigung des ultradünnen Endoskops auf ein Mindestmaß reduziert. Darüber hinaus hängt das Endoskop nicht "durch"; dies ist bei der Handhabung ultradünner Endoskope besonders störend und eine häufige Ursache für Beschädigungen.

Zusätzlich zur Verwendung eines Kopfhalters kann die Beobachtungseinrichtung selbstverständlich bei Bedarf in gleicher Weise wie ein Operationsmikroskop "aufgeständert" werden, wenn vorübergehend die Benutzung des Kopfhalters stört (Anspruch 25).

Wie bereits ausgeführt, kann die erfindungsgemäße Vorrichtung sowohl im technischen als auch im medizinischen Bereich universell eingesetzt werden. Das Endoskop kann dabei in an sich bekannter Weise in einem Schaft geführt sein. Dieser Schaft kann nicht nur wenigstens einen Kanal für ein Operationsinstrument aufweisen, sondern auch für die Führung eines zusätzlichen Lichtleiters dienen, der das Objektfeld beleuchtet (Ansprüche 20 bis 22).

Im medizinischen Bereich sind nicht nur bei minimalinvasiven oder mikrochirurgischen Operationen Einsatzmöglichkeiten vorhanden, sondern auch bei der Untersuchung von Augen und zur Durchführung von Eingriffen am menschlichen oder tierischen Auge. Bei der Untersuchung bzw. dem Eingriff wird das Endoskop unmittelbar neben dem Auge, beispielsweise im Bereich des Tränensacks positioniert, oder in das Auge, wie beispielsweise den Glaskörper eingeführt. Wie bei endoskopischen Untersuchungen üblich, ist eine Beleuchtungseinrichtung vorgesehen, deren Licht in einen in dem Endoskop angeordneten Lichtleiter eingekoppelt wird, der das Objektfeld des Endoskopobjektivs beleuchtet.

Bei Eingriffen am Auge ist bevorzugt an dem Kopfband oder an der Binokularlupe bzw. dem Fernrohr eine Beleuchtungseinrichtung angebracht sein, die das zu untersuchende Auge beleuchtet. Damit hat die Untersuchungsperson die Möglichkeit, mit dem einen Auge das zu untersuchende bzw. zu behandelnde Auge zu beobachten, bei dem das Endoskop angeordnet ist bzw. in das das Endoskop eingesetzt ist. Ohne das Endoskop entfernen zu müssen, kann sie dann die Umgebung, beispielsweise den "Einstich" des Endoskops in das Auge oder andere Bereich dieses Auges inspizieren. Die Beleuchtungseinrichtung kann dabei zwischen den beiden Strahlengängen der Binokularlupe angeordnet sein, so daß auch bei binokularer Betrachtung des Auges ohne angeflanschtes Endoskop das Objektfeld vollständig ausgeleuchtet wird.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, in der zeigen:
- Fig. 1: eine schematische perspektivische Ansicht eines ersten Ausführungsbeispiels der Erfindung,
- Fig. 2: eine erfindungsgemäß optional vorgesehene Schutzhülse,
- Fig. 3: ein zweites Ausführungsbeispiel der Erfindung, und
- Fig. 4: ein drittes Ausführungsbeispiel der Erfindung.

### Darstellung von Ausführungbeispielen

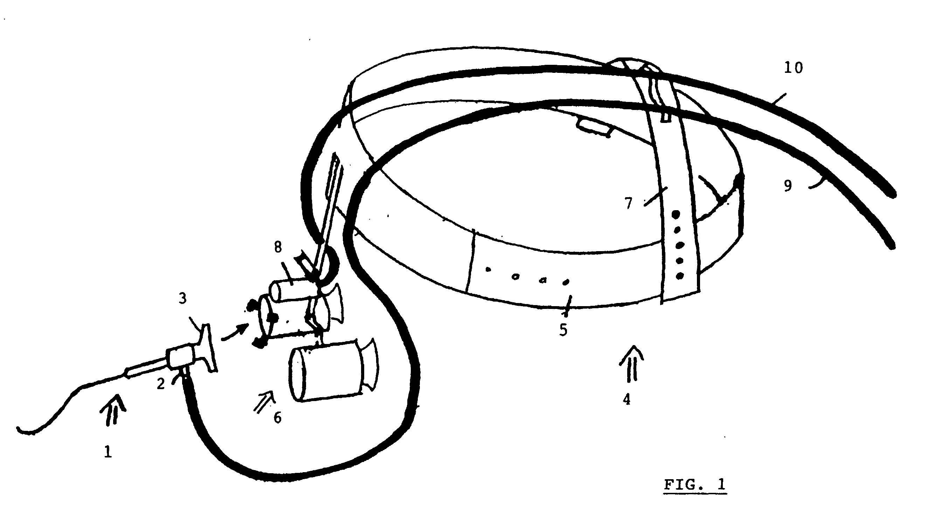

Fig. 1 zeigt ein erstes Ausführungsbeispiel einer erfindungsgemäßen Vorrichtung zur Untersuchung von Hohlräumen insbesondere im menschlichen oder tierischen Körper.

Bestandteil der erfindungsgemäßen Vorrichtung ist ein ultradünnes Endoskop 1, das einen nicht im einzelnen dargestellten Lichtleiter mit einem Lichtleiteranschluß 2 sowie am - nicht dargestellten distalen Ende - ein Objektiv aufweist. Das von dem Objektiv erzeugte Bild wird von einem Bildweiterleiter zum in Fig. 1 dargestellten proximalen Ende des Endoskops geleitet, so daß es durch ein Okular 3 betrachtet werden kann.

Bei dem gezeigten Ausführungsbeispiel ist das Endoskop 1 ein flexibles Endoskop; dementsprechend besteht der Bildweiterleiter bevorzugt aus einem geordneten Glasfaserbündel.

Erfindungsgemäß ist ein Kopfband 4 vorgesehen, das in an sich bekannter Weise aus einem ringförmigen Teil 5 und einem Halteband 7 besteht. Der Durchmesser des ringförmigen Teils 5 und die Länge des Haltebandes 7 sind einstellbar. Selbstverständlich sind aber auch andere Ausführungen möglich.

An dem Kopfband 4 ist eine an sich bekannte Binokularlupe 6 mit den üblichen Einstellmöglichkeiten für Neigung, Pupillenabstand und gegebenenfalls Fokussierung angebracht. Ein Strahlengang der Binokularlupe 6 - bei dem gezeigten Ausführungsbeispiel der von dem Träger des Kopfbandes aus gesehene rechte Durchblick - weist auf seiner dem Einblick abgewandten Seite eine Kupplung auf, die das Anflanschen des Okulartrichters 3 des ultradünnen Endoskops 1 erlaubt. Für diese Kupplung können Kupplungstypen verwendet werden, wie sie beispielsweise für das Anflanschen von Endoskopen an Videokameras bekannt sind. Damit ist das Okular nicht nur vor dem Auge des Kopfband-Trägers positioniert und folgt jeder Kopfbewegung, ohne daß der Kopfband-Träger das Endoskop halten bzw. bei Verwendung einer herkömmlichen Haltevorrichtung laufend die Haltevorrichtung neu einstellen müßte, sondern es wird auch das Okularbild vergrößert. Dies erleichtert nicht nur die Betrachtung des bei ultradünnen Endoskopen sehr kleinen Okularbildes, sondern erspart auch in vielen Fällen die zusätzliche bzw. ergänzende Verwendung eines Operationsmikroskopes.

Das Kopfband kann ferner dazu benutzt werden, eine Beleuchtungseinrichtung 8 zu halten, die die Umgebung beleuchtet. Die Beleuchtungseinrichtung 8, die mit einer nicht im einzelnen dargestellten Kondensorlinse versehen ist, ist zwischen dem linken und dem rechten Strahlengang der Binokularlupe 6 angeordnet.

Zur Licht-Versorgung des Lichtleiters des Endoskops und der Beleuchtungseinrichtung sind Lichtleiter 9 und 10 vorgesehen, die am Kopfband 4 und insbesondere am Halteband 7 über geeignete Halterungen geführt sind.

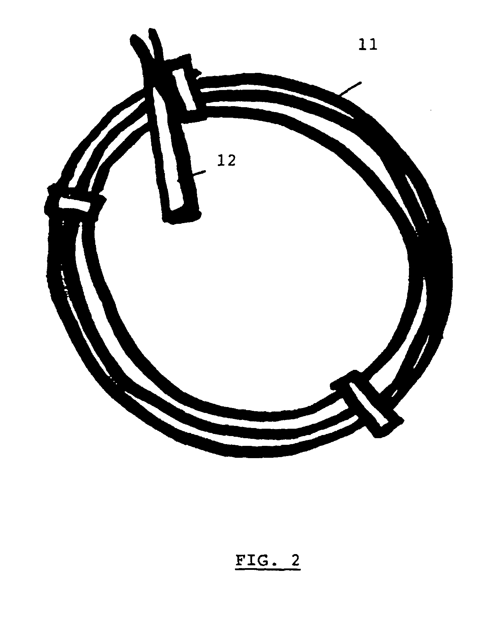

Fig. 2 zeigt eine Schutzhülse 11 für das Endoskop 1, aus der das flexible Endoskop herausziehbar ist. Die Schutzhülse 11 ist spiralförmig ausgebildet. Ferner ist eine Klammer 12 vorgesehen, mit der die Schutzhülse an der Kleidung der Untersuchungsperson befestigbar ist.

Da das ultradünne Endoskop 1 aus der Schutzhülse 11 immer nur soweit herausgezogen wird, wie es für den jeweiligen Untersuchungsvorgang benötigt wird, wird die Gefahr einer Beschädigung des ultradünnen Endoskops auf ein Mindestmaß reduziert. Darüberhinaus hängt das Endoskop nicht "durch"; dieses Durchhängen ist bei der Handhabung ultradünner Endoskope besonders störend und eine häufige Ursache für Beschädigungen.

Im Rahmen der Erfindung sind gegenüber dem in Fig. 1 dargestellten Ausführungsbeispiels selbstverständlich die verschiedensten Modifikationen möglich:

So kann das Kopfband anders gestaltet sein als in der Zeichnung dargestellt. Anstelle eines Kopfbandes kann auch ein anderer Kopfträger verwendet werden.

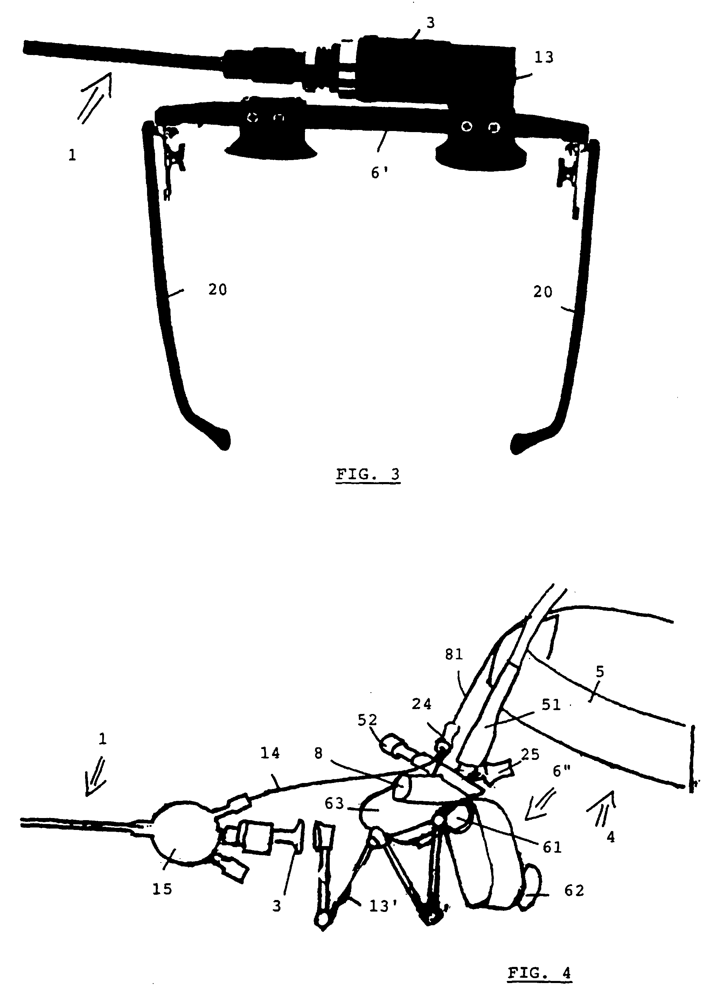

Eine entsprechende Möglichkeit ist in Fig. 3 dargestellt: Bei dem in dieser Figur dargestellten zweiten Ausführungsbeispiel wird anstelle der Binokular-Lupe 7 als Betrachtungseinheit eine Brille 6' bzw. eine "Brillen-ähnliche" Fassung mit Brillenbügeln 20 verwendet. Bei diesem Ausführungsbeispiel ist ferner das Okular 3 des Endoskops 1 nicht direkt an dem Gläserring der Brillenfassung, d.h. der Austrittsöffnung der Betrachtungseinheit 6' angebracht, sondern aufgrund der Verwendung eines Umlenkprismas 13 versetzt und unter einem Winkel von 90° angeordnet werden. Der Okularteil des Endoskops 1 stützt sich auf dem Nasenrücken der Untersuchungsperson ab.

In Fig. 4 ist eine erfindungsgemäße Vorrichtung zur Untersuchung des Auges sowie zur Durchführung von Eingriffen am Auge dargestellt. Die Vorrichtung weist ein Endoskop 1 auf, das neben einem nicht dargestellten Auge positionierbar oder in das Augeninnere einführbar ist. Ferner ist eine von dem Endoskop 1 getrennte Beobachtungseinrichtung vorgesehen. Bei dem gezeigten Ausführungsbeispiel ist die Beobachtungseinrichtung ein Prismenfernrohr 6".

Der Okulartrichter 3 des Endoskops 1 ist über eine Gliederoptik 13' mit einer Ausblicköffnung 61 des Fernrohrs 6" verbunden. Damit kann durch das der Ausblicköffnung 61 zugeordnete Okular 62 des Fernrohrs 6" das Okularbild des Endoskops vergrößert betrachtet werden.

Zwischen den beiden Teilen des Binokular-Prismenfernrohrs 6 ist eine Beleuchtungseinrichtung 8 vorgesehen, die die Umgebung beleuchtet, die durch den anderen Strahlengang des Prismenfernrohrs 6" betrachtet werden kann. Der zweite Strahlengang ist schematisch mit dem Bezugszeichen 63 bezeichnet.

Ferner ist eine nicht dargestellte Lichtquelle vorgesehen, deren Licht über einen Lichtleiter 81 in die Beleuchtungseinrichtung 8 eingekoppelt wird. Das Licht der Lichtquelle wird ferner in einen dünnen Lichtleiter 14 eingekoppelt, der zur Beleuchtung des Gesichtsfeldes des Endoskops dient. Der Lichtleiter 81 ist durch eine eine Führung 24 am Prismenfernrohr 6" geführt. Eine weitere Führung 25 dient zum Halten eines weiteren Lichtleiters oder eines dünnen flexiblen Endoskops.

Das Endoskop 1 ist in an sich bekannter Weise in einem Schaft 15 geführt, in dem auch der Lichtleiter 14 geführt wird.

Der Schaft 15 kann einen oder mehrere Kanäle für Operationsinstrumente aufweisen.

Das Prismenfernrohr 6" ist wiederum an einem Kopfband 5 über einen Halterung 51 und eine Einstellschraube 52 zur Verstellung der Neigung angebracht.

Gegenüber den vorstehend beschriebenen Ausführungsbeispielen sind selbstverständlich die verschiedensten Abwandlungen im Rahmen der Erfindung möglich:

Alternativ kann auch das Endoskop nicht direkt an der Austrittsöffnung der Lupe, sondern versetzt und unter einem Winkel angeordnet werden. Der Strahlengang eines Durchblicks des Betrachtungssystems, wie der Binokularlupe kann dann durch ein vor die Austrittsöffnung der Binokularlupe schwenkbare Umlenkprisma so umgelenkt werden, daß anstelle der Umgebung das Okularbild durch die Lupe betrachtet werden kann.

Die erfindungsgemäß zur Betrachtung des Okularbildes eines Endoskops und zusätzlich zur Beobachtung der Umgebung vorgesehene binokulare Betrachtungseinheit kann selbstverständlich auch dazu benutzt werden, die Okularbilder von Endoskopen zu betrachten, die nicht ultradünn im vorstehend definierten Sinne sind, also beispielsweise von starren Endoskopen, die einen Durchmesser von 4 mm haben.

## Patentansprüche

1. Vorrichtung zur Untersuchung von Hohlräumen, insbesondere im menschlichen oder tierischen Körper, mit
- einem Endoskop, das einen Lichtleiter zur Beleuchtung des Hohlraums sowie ein Objektiv, einen Bildweiterleiter für das Bild des Objektivs und ein Okular (3) zur Betrachtung des weitergeleiteten Bilds aufweist, und
- einem Kopfhalter (4), an dem das Okular (3) des Endoskops (1) derart angebracht ist, daß es vor einem Auge der Untersuchungsperson positioniert ist,
**dadurch gekennzeichnet, daß** an dem Kopfhalter (4) ein Binokular-Betrachtungssystem (6) angebracht ist,
dass der Okular (3) des Endoskops (1) monokular mit einem Strahlengang des Betrachtungssystems verbunden ist und
daß das Betrachtungssystem das Okularbild für die Untersuchungsperson vergrößert.

2. Vorrichtung zur Untersuchung von Hohlräumen, insbesondere im menschlichen oder tierischen Körper, mit
- einem Endoskop, das einen Lichtleiter zur Beleuchtung des Hohlraums sowie ein Objektiv, einen Bildweiterleiter für das Bild des Objektivs und ein Okular (3) zur Betrachtung des weitergeleiteten Bilds aufweist, und
- einem Kopfhalter (4), an dem eine Gliederoptik (13') derart angebracht ist, daß sie vor einem Auge der Untersuchungsperson positioniert ist,
**dadurch gekennzeichnet, daß** an dem Kopfhalter (4) ein Binokular-Betrachtungssystem (6) angebracht ist,
daß das Okular (3) des Endoskops (1) mit dem Betrachtungssystem (6) über die Gliederoptik (13') verbunden ist,
daß das Okular (3) des Endoskops (1) monokular mit einem Strahlengang des Betrachtungssystems verbunden ist und
daß das Betrachtungssystem das Okularbild für die Untersuchungsperson vergrößert.

3. Vorrichtung nach Anspruch 2,
**dadurch gekennzeichnet, daß** eine Spirale die Gliederoptik umgibt.

4. Vorrichtung nach Anspruch 2,
**dadurch gekennzeichnet, daß** wenigstens ein Lichtleitkabel zur Beleuchtung der Umgebung in der Spirale geführt ist.

5. Vorrichtung nach einem der Ansprüche 1 oder 2,
**dadurch gekennzeichnet, daß** der Kopfhalter in Art einer Brillenfassung mit geeigneten Gläsern, einer Binokularlupe (6) oder eines Fernrohrs und insbesondere eines Prismenfernrohrs ausgebildet ist, die das Betrachtungssystem bilden.

6. Vorrichtung nach Anspruch 5,
**dadurch gekennzeichnet, daß** die Bügel der Brillenfassung den Kopfhalter bilden.

7. Vorrichtung nach einem der Ansprüche 1 bis 6,
**dadurch gekennzeichnet, daß** der Kopfhalter (4) ein Kopfband (5,7) aufweist.

8. Vorrichtung nach einem der Ansprüche 1, 5 bis 7,
**dadurch gekennzeichnet, daß** das Okular (3) des Endoskops (1) ohne zwischengeschaltetes optisches System mit dem Betrachtungssystem (6) über eine geeignete Kupplung verbunden ist.

9. Vorrichtung nach einem der Ansprüche 1, 5 bis 8,
**dadurch gekennzeichnet, daß** das Okular (3) des Endoskops mit dem Betrachtungssystem über ein Strahl-Umlenkelement (13) verbunden ist.

10. Vorrichtung nach Anspruch 9,
**dadurch gekennzeichnet, daß** das Umlenkelement den Strahlengang um 90° umlenkt.

11. Vorrichtung nach Anspruch 10,
**dadurch gekennzeichnet, daß** sich der Okularteil des Endoskops auf dem Nasenrücken der Untersuchungsperson abstützt.

12. Vorrichtung nach einem der Ansprüche 1 bis 11,
**dadurch gekennzeichnet, daß** ein optisches System vorgesehen ist, das ein strahlumlenkendes Element, wie ein Umlenkprisma, in den Strahlengang vor einer Austrittsöffnung einbringt, so daß zwischen binokularer Beobachtung der Umgebung und Betrachtung des Okularbildes des Endoskops sowie gegebenenfalls gleichzeitiger Betrachtung der Umgebung umgeschaltet werden kann.

13. Vorrichtung nach einem der Ansprüche 1 bis 12,
**dadurch gekennzeichnet, daß** an dem Kopfhalter (4) und/oder dem Betrachtungssystem eine Beleuchtungseinrichtung (8) angebracht ist, welche die Umgebung beleuchtet.

14. Vorrichtung nach Anspruch 13,
**dadurch gekennzeichnet, daß** die Beleuchtungseinrichtung (8) zwischen den beiden Strahlengängen bzw. den beiden Ausblicköffnungen des Betrachtungssystems (6) an diesem angebracht ist.

15. Vorrichtung nach Anspruch 13 oder 14,
**dadurch gekennzeichnet, daß** die Beleuchtungseinrichtung einen Lichtleiter aufweist, der sie mit einer Lichtquelle verbindet.

16. Vorrichtung nach Anspruch 15,
**dadurch gekennzeichnet, daß** der Lichtleiter für die Beleuchtungseinrichtung (8) am Kopfhalter und insbesondere am Kopfband (4) geführt ist.

17. Vorrichtung nach einem der Ansprüche 1 bis 16,
**dadurch gekennzeichnet, daß** ein Lichtleit-Kabel, welches das Licht von der Beleuchtungseinrichtung in den in dem Endoskop angeordneten Lichtleiter einkoppelt, an der Betrachtungssystem und/oder am Kopfhalter geführt ist.

18. Vorrichtung nach einem der Ansprüche 1 bis 17,
**dadurch gekennzeichnet, daß** das Endoskop ein ultradünnes Endoskop ist.

19. Vorrichtung nach Anspruch 18,
**dadurch gekennzeichnet, daß** das ultradünne Endoskop (1) ein flexibles Endoskop ist.

20. Vorrichtung nach einem der Ansprüche 1 bis 19,
**dadurch gekennzeichnet, daß** das Endoskop (1) in einem Schaft (5) geführt ist.

21. Vorrichtung nach Anspruch 20,
**dadurch gekennzeichnet, daß** in dem Schaft ein zusätzlicher Lichtleiter für die Beleuchtung des Objektfeldes des Endoskops geführt ist.

22. Vorrichtung nach Anspruch 21,
**dadurch gekennzeichnet, daß** in dem Schaft wenigstens ein Kanal für ein Operationsinstrument vorgesehen ist.

23. Vorrichtung nach einem der Ansprüche 19 bis 22,
**dadurch gekennzeichnet, daß** der Schaft eine Schutzhülse (11) für das Endoskop (1) ist, aus der das Endoskop herausziehbar ist.

24. Vorrichtung nach Anspruch 23,
**dadurch gekennzeichnet, daß** die Schutzhülse (11) an der Kleidung der Untersuchungsperson befestigbar ist.

25. Vorrichtung nach einem der Ansprüche 1 bis 24,
**dadurch gekennzeichnet, daß** zusätzlich ein Ständer für das Betrachtungssystem vorgesehen ist.

## Claims

1. Device for examining cavities, particularly in a human or animal body, comprising
- an endoscope having a light guide for illuminating the cavity, and also an objective lens, an image transmitting means for the image from the objective lens, and an eyepiece (3) for observing the transmitted image; and
- a head-borne holder (4) to which the eyepiece (3) of the endoscope (1) is attached in such manner that it is positioned in front of one eye of an examining person;
**characterized in that** a binocular observation system (6) is attached to the head-borne holder (4);
that the eyepiece (3) of the endoscope (1) is linked to one beam path of the observation system in a monocular arrangement; and
that the observation system magnifies the eyepiece-image for an examining person.

2. Device for examining cavities, particularly in a human or animal body, comprising
- an endoscope having a light guide for illuminating the cavity, and also an objective lens, an image transmitting means for the image from the objective lens, and an eyepiece (3) for observing the transmitted image; and
- a head-borne holder (4) to which a linked optical system (13') is attached in such manner that it is positioned in front of one eye of an examining person;
**characterized in that** a binocular observation system (6) is attached to the head-borne holder (4);
that the eyepiece (3) of the endoscope (1) is linked to the observation system via the linked optical system;
that the eyepiece (3) of the endoscope (1) is linked to one beam path of the observation system in a monocular arrangement; and
that the observation system magnifies the eyepiece-image for an examining person.

3. Device according to claim 2,
**characterized in that** a spiral surrounds the linked optical system.

4. Device according to claim 2,
**characterized in that** at least one light guide cable for illuminating the surroundings is guided in the spiral.

5. Device according to any one of claims 1 or 2,
**characterized in that** the head-borne holder is designed in the manner of a spectacle frame with suitable lenses, a binocular magnifier (6), or a telescope, in particular a prism telescope, which form the observation system.

6. Device according to claim 5,
**characterized in that** the spectacle temple arms of the spectacle frame form the head-borne holder.

7. Device according to any one of claims 1 to 6,
**characterized in that** the head-borne holder (4) comprises a head-band (5, 7).

8. Device according to any one of claims 1, 5 to 7,
**characterized in that** the eyepiece (3) of the endoscope (1) is linked to the observation system (6) via suitable coupling means without an interposed optical system.

9. Device according to any one of claims 1, 5 to 8,
**characterized in that** the eyepiece (3) of the endoscope (1) is linked to the observation system via a beam-deflecting component (13).

10. Device according to claim 9,
**characterized in that** the deflecting component deflects the beam path by 90°.

11. Device according to claim 10,
**characterized in that** the eyepiece portion of the endoscope is supported on the bridge of the nose of the examining person.

12. Device according to any one of claims 1 to 11,
**characterized in that** an optical system is provided which inserts a beam-deflecting component such as a deflecting prism into the beam path in front of an exit aperture, so that a switch-over may be performed between a binocular observation of the surroundings and an observation of the eyepiece image of the endoscope, and possibly also a simultaneous observation of the surroundings.

13. Device according to any one of claims 1 to 12,
**characterized in that** an illumination means (8) for illuminating the surroundings is attached to the head-bome holder (4) and/or the observation system.

14. Device according to claim 13,
**characterized in that** the illumination means (8) is attached to the observation system (6) between both beam paths or outlook apertures thereof.

15. Device according to claim 13 or 14,
**characterized in that** the illumination means comprises a light guide which connects it to a light source.

16. Device according to claim 15,
**characterized in that** the light guide for the illumination means (8) is carried on the head-borne holder and, in particular, the head-band (4).

17. Device according to any one of claims 1 to 16,
**characterized in that** a light-guide cable which couples the light from the illumination means into the light guide disposed in the endoscope, is carried on the observation system and/or the head-borne holder.

18. Device according to any one of claims 1 to 17,
**characterized in that** the endoscope is an ultra-thin endoscope.

19. Device according to claim 18,
**characterized in that** the ultra-thin endoscope (1) is a flexible endoscope.

20. Device according to any one of claims 1 to 19,
**characterized in that** the endoscope (1) is guided within a shall (5).

21. Device according to claim 20,
**characterized in that** an additional light guide for illumination of the object field of the endoscope is carried within the shaft.

22. Device according to claim 21,
**characterized in that** at least one passage for an operating instrument is provided within the shaft.

23. Device according to any one of claims 19 to 22,
**characterized in that** the shall is a protective sheath (11) for the endoscope (1), from which the endoscope may be withdrawn.

24. Device according to claim 23,
**characterized in that** the protective sheath (11) is adapted to be fastened to the clothing of the examining person.

25. Device according to any one of claims 1 to 24,
**characterized in that** additionally a stand is provided for the observation system.

## Revendications

1. Dispositif d'examen de cavités, en particulier dans l'organisme humain ou animal, comprenant
- un endoscope présentant un conduit de lumière pour éclairer la cavité ainsi qu'un objectif, un dispositif de transmission de l'image de l'objectif et un oculaire (3) pour observer l'image transmise, et
- un dispositif de fixation sur la tête (4) sur lequel l'oculaire (3) de l'endoscope (1) est fixé de telle sorte qu'il est positionné devant un oeil de la personne qui effectue l'examen,
**caractérisé en ce que** qu'un système d'observation binoculaire (6) est fixé sur le dispositif de fixation sur la tête (4), **en ce que** l'oculaire (3) de l'endoscope (1) est relié de manière monoculaire avec une trajectoire de faisceau lumineux du système d'observation et **en ce que** le système d'observation agrandit l'image de l'oculaire pour la personne qui effectue l'examen.

2. Dispositif d'examen de cavités, en particulier dans l'organisme humain ou animal, comprenant
- un endoscope présentant un conduit de lumière pour éclairer la cavité ainsi qu'un objectif, un dispositif de transmission de l'image de l'objectif et un oculaire (3) pour observer l'image transmise, et
- un dispositif de fixation sur la tête (4) sur lequel une optique articulée (13') est fixée de telle sorte qu'elle est positionnée devant un oeil de la personne qui effectue l'examen,
**caractérisé en ce que** qu'un système d'observation binoculaire (6) est fixé sur le dispositif de fixation sur la tête (4), **en ce que** l'oculaire (3) de l'endoscope (1) est relié au système d'observation (6) par l'intermédiaire de l'optique articulée (13'), **en ce que** l'oculaire (3) de l'endoscope (1) est relié de manière monoculaire avec une trajectoire de faisceau lumineux du système d'observation et **en ce que** le système d'observation agrandit l'image de l'oculaire pour la personne qui effectue l'examen.

3. Dispositif selon la revendication 2, **caractérisé en ce qu'**une spirale entoure l'optique articulée.

4. Dispositif selon la revendication 2, **caractérisé en ce qu'**au moins un câble de transmission de la lumière est guidé dans la spirale afin d'éclairer l'environnement.

5. Dispositif selon l'une des revendications 1 ou 2, **caractérisé en ce que** le dispositif de fixation sur la tête se présente sous la forme d'une monture de lunettes avec des verres appropriés, une loupe binoculaire (6) ou un télescope, en particulier un télescope à prismes, formant le système d'observation.

6. Dispositif selon la revendication 5, **caractérisé en ce que** les branches de la monture de lunettes forment le dispositif de fixation sur la tête.

7. Dispositif selon l'une des revendications 1 à 6, **caractérisé en ce que** le dispositif de fixation sur la tête (4) présente une bande serre-tête (5, 7).

8. Dispositif selon l'une des revendications 1 et 5 à 7, **caractérisé en ce que** l'oculaire (3) de l'endoscope (1) est relié au système d'observation (6), sans système optique intercalé, par l'intermédiaire d'un accouplement approprié.

9. Dispositif selon l'une des revendications 1 et 5 à 8, **caractérisé en ce que** l'oculaire (3) de l'endoscope (1) est relié au système d'observation par l'intermédiaire d'un élément de déviation du faisceau lumineux (13).

10. Dispositif selon la revendication 9, **caractérisé en ce que** l'élément de déviation dévie la trajectoire du faisceau lumineux de 90°.

11. Dispositif selon la revendication 10, **caractérisé en ce que** la partie oculaire de l'endoscope s'appuie sur l'arête du nez de la personne qui effectue l'examen.

12. Dispositif selon l'une des revendications 1 à 11, **caractérisé en ce qu'**il est prévu un système optique qui introduit un élément de déviation du faisceau lumineux, par exemple un prisme déviateur, dans la trajectoire du faisceau avant un orifice de sortie de façon à permettre de passer de l'observation binoculaire de l'environnement à l'observation de l'image de l'oculaire de l'endoscope ainsi que, le cas échéant, à l'observation simultanée de l'environnement.

13. Dispositif selon l'une des revendications 1 à 12, **caractérisé en ce qu'**un dispositif d'éclairement (8) qui éclaire l'environnement est fixé sur le dispositif de fixation sur la tête (4) et/ou sur le système d'observation.

14. Dispositif selon la revendication 13, **caractérisé en ce que** le dispositif d'éclairement (8) est fixé sur le système d'observation (6) entre les deux trajectoires du faisceau lumineux ou entre les deux orifices de visée de celui-ci.

15. Dispositif selon la revendication 13 ou la revendication 14, **caractérisé en ce que** le dispositif d'éclairement présente un conduit de lumière qui le relie à une source lumineuse.

16. Dispositif selon la revendication 15, **caractérisé en ce que** le conduit de lumière du dispositif d'éclairement (8) est guidé sur le dispositif de fixation sur la tête et notamment sur le serre-tête (4).

17. Dispositif selon l'une des revendications 1 à 16, **caractérisé en ce qu'**un câble de transmission de la lumière couplant la lumière venant du dispositif d'éclairement au conduit de lumière agencé dans l'endoscope est guidé sur le système d'observation et/ou le dispositif de fixation sur la tête.

18. Dispositif selon l'une des revendications 1 à 17, **caractérisé en ce que** l'endoscope est un endoscope ultrafin.

19. Dispositif selon la revendication 18, **caractérisé en ce que** l'endoscope ultrafin (1) est un endoscope souple.

20. Dispositif selon l'une des revendications 1 à 19, **caractérisé en ce que** l'endoscope (1) est guidé dans un fût (15).

21. Dispositif selon la revendication 20, **caractérisé en ce qu'**un conduit de lumière supplémentaire est guidé dans le fût afin d'éclairer le champ de l'objet de l'endoscope.

22. Dispositif selon la revendication 21, **caractérisé en ce qu'**il est prévu dans le fût au moins un canal pour un instrument opératoire.

23. Dispositif selon l'une des revendications 19 à 22, **caractérisé en ce que** le fût est une gaine de protection (11) pour l'endoscope (1), de laquelle l'endoscope peut être retiré.

24. Dispositif selon la revendication 23, **caractérisé en ce que** la gaine de protection (11) peut être fixée sur les vêtements de la personne qui effectue l'examen.

25. Dispositif selon l'une des revendications 1 à 24, **caractérisé en ce qu'**il est prévu en plus un statif pour le système d'observation.
